# EUROPEAN PATENT APPLICATION

(11) **EP 4 692 311 A1**
(43) Date of publication of application: **11.02.2026**
(21) Application number: 25788010.4
(22) Date of filing: 13.05.2025
(51) Int. Cl.: C12N 1/20, A61K 35/747, A61P 11/00, A61P 31/04, C12R 1/23, C12R 1/25

(54) **COMPOSITE PROBIOTIC FOR PREVENTING AND TREATING INFECTIOUS PNEUMONIA AND USE THEREOF**

(30) Priority: 25.06.2024 CN 202410823064
(71) Applicant: Wecare Probiotics Co., Ltd., Suzhou, Jiangsu 215200 (CN)
(72) Inventor: FANG, Shuguang, Suzhou, Jiangsu 215200 (CN); WU, Zhiyi, Suzhou, Jiangsu 215200 (CN); DONG, Yao, Suzhou, Jiangsu 215200 (CN); GAI, Zhonghui, Suzhou, Jiangsu 215200 (CN); ZHU, Jianguo, Suzhou, Jiangsu 215200 (CN)
(74) Representative: Lanchava, Bakuri
(86) International application number: PCT/CN2025/094611
(87) International publication number: WO 2026/001390

(57) **Abstract**

The present invention involves a compound probiotic for prevention and treatment of infectious pneumonia and use thereof, and the compound probiotic for prevention and treatment of infectious pneumonia consists of the *Lactobacillus acidophilus* LA85 strain and the *Lactobacillus plantarum Lp05* strain. The two strains can cooperate with each other for mutual promotion, thus synergizing the effect of preventing and treating infectious pneumonia. Compared with the intervention method with only one strain, the compounding of the two strains is significantly more effective in the prevention and treatment of infectious pneumonia under the same dosage. At the same time, both strains are probiotics, so the product is safer and resistance to the product will not be easily generated.

## Description

### Technical Field

The present invention belongs to the technical field of microorganism, and relates to a compound probiotic for prevention and treatment of infectious pneumonia and use thereof.

### Background Art

Some bacteria like Streptococcus pneumoniae, Pseudomonas aeruginosa and Staphylococcus aureus are important pathogens for pneumonia, while continuous inflammatory response produces excessive reactive oxygen, which can directly destroy nucleic acids, proteins and lipids, resulting in an irreversible damage to the body, and, reactive oxygen and inflammatory response will initiate a cycle of mutual promotion, spreading throughout the body, thus leading to systemic inflammatory syndrome. For the treatment of bacterial pneumonia, generally, antibiotics are delivered directly to the infected area by inhalation administration. Although conventional medication can alleviate some symptoms, it often causes significant side effects; therefore, it is urgent to find a safer and more effective alternative treatment method.

In recent years, with the in-depth study on the mechanism of gut-lung axis interaction, probiotics, as natural microorganisms that regulate the host's immune response and improve the balance of the intestinal microbial community, have been gradually revealed for their potential in prevention and treatment of pneumonia. Gut microbes are believed to play a key role in regulating the host's local and systemic immune responses, and through gut-lung axis interactions, a change in gut microbes affects the immune environment of the lungs, and in turn affects the resistance to respiratory pathogens.

The use of probiotics in the treatment of infectious pneumonia is still in a preliminary stage, and there are few strategies for the prevention and treatment of infectious pneumonia. Therefore, developing more probiotics for control of infectious pneumonia can not only provide new ideas and methods for the treatment of infectious pneumonia, but also help to deeply understand the role of the gut-lung axis in diseases, thus providing scientific basis and technical support for the prevention and treatment of respiratory tract infectious diseases.

### Summary of the Invention

In view of the deficiencies of the prior art, the present invention aims to provide a compound probiotic for prevention and treatment of infectious pneumonia and use thereof.

For attaining the purpose of the present invention, the present invention adopts the following technical solutions:
In a first aspect, the present invention provides a compound probiotic for the prevention and treatment of infectious pneumonia, and the compound probiotic consists of the *Lactobacillus acidophilus* LA85 strain preserved under GMCC No. 1.12735 and the *Lactobacillus plantarum* Lp05 strain preserved under CGMCC No. 23547.

For the present invention, a brand-new probiotic compounding method and a brand-new strategy for prevention and treatment of infectious pneumonia have been creatively developed, that is, after the *Lactobacillus acidophilus* LA85 strain and the *Lactobacillus plantarum* Lp05 strain are compounded for combination use, it is found that the two can cooperate with each other for mutual promotion, thus synergizing the effect of preventing and treating infectious pneumonia. This is specifically manifested in the following aspects: (1) significantly inhibiting the growth of infectious microorganisms in the lungs and increasing the removal rate of infectious microorganisms; (2) significantly alleviating the pulmonary inflammatory response; (3) significantly improving the survival rate of model mice with infectious pneumonia. The compounding of the two strains is significantly more effective in the prevention and treatment of infectious pneumonia than the intervention method with any of the two strains alone under the same dosage. At the same time, both strains are probiotics, and so the product is safer and resistance to the product will not be easily generated.

Preferably, the viable count ratio of the LA85 strain to the Lp05 strain is ranged from 1:10 to 10:1, e.g., 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, etc.. Other specific values for the ratio can also be selected if within the above numerical range, which will not be detailed here.

In a second aspect, the present invention provides a probiotic preparation for the prevention and treatment of infectious pneumonia, and the strains in the probiotic preparation comprise the compound probiotic described in the first aspect.

Preferably, the viable content of the LA85 strain and the Lp05 strain in the probiotic preparation is not less than 1×10⁹ CFU/g or 1×10⁹ CFU/mL respectively, for example, 1×10⁹ CFU/g (CFU/mL), 1×10¹⁰ CFU/g (CFU/mL), 5×10¹⁰ CFU/g (CFU/mL), 1×10¹¹ CFU/g (CFU/mL), 3×10¹¹ CFU/g (CFU/mL), 5× 10¹¹ CFU/g (CFU/mL), 1×10¹² CFU/g (CFU/mL), 1×10¹³ CFU/g (CFU/mL), etc. Other specific values in this numerical range can also be selected, which will not be detailed here.

Preferably, the dosage forms of the probiotic preparation include solution, freeze-dried powder, capsules, tablets or granules. The dosage forms of the probiotic preparation in this invention are not limited, and include the most commonly used solution, freeze-dried powder, or further-prepared capsules, tablets or granules.

Preferably, solution is adopted as the dosage form of the probiotic preparation, which is prepared by the following method:
Inoculate The LA85 strain and Lp05 strain in the media respectively for activation and fermentation culture, so as to obtain the fermentation broths. Centrifuge the fermentation broths respectively, and then resuspend with solvent to obtain LA85 bacterial suspension and Lp05 bacterial suspension. Mix LA85 bacterial suspension and Lp05 bacterial suspension based on the viable count ratio, to obtain the probiotic preparation.

Preferably, freeze-dried powder is adopted as the dosage form of the probiotic preparation, which is prepared by the following method:
Inoculate the LA85 strain and Lp05 strain in the media respectively for activation and fermentation culture, so as to obtain the fermentation broths. Centrifuge the fermentation broths respectively, then mix with a protective agent for freeze-drying, to obtain LA85 bacterial powder and Lp05 bacterial powder. Mix LA85 bacterial powder and Lp05 bacterial powder based on the viable count ratio, to obtain the probiotic preparation.

In a third aspect, the present invention provides the use of compound probiotic according to the first aspect or probiotic preparation according to the second aspect in preparation of drugs for the prevention, alleviation or treatment of infectious pneumonia.

Preferably, the drugs also contain excipients.

Preferably, the excipients include any one or at least two of fillers, binders, wetting agents, disintegrants, emulsifiers, co-solvents, solubilizers, osmotic pressure regulators, colorants, pH adjusters, antioxidants, bacteriostatic agents or buffers.

Compared with the prior art, the present application has the following beneficial effects:
For the invention, a brand-new probiotic compounding method and a brand-new strategy for the prevention and treatment of infectious pneumonia have been creatively developed, that is, after the *Lactobacillus acidophilus* LA85 strain and the *Lactobacillus plantarum* Lp05 strain are compounded for combination use, it is found that the two can cooperate with each other for mutual promotion, thus synergizing the effect of preventing and treating infectious pneumonia. This is specifically manifested in the following aspects: (1) significantly inhibiting the growth of infectious microorganisms in the lungs and increasing the removal rate for infectious microorganisms; (2) significantly alleviating the pulmonary inflammatory response; (3) significantly improving the survival rate of model mice with infectious pneumonia. Compared with the intervention method with only one strain, the compounding of the two strains is significantly more effective in the prevention and treatment of infectious pneumonia under the same dosage. At the same time, both strains are probiotics, so the product is safer and the resistance to the product will not be easily generated.

According to the Classified Nomenclature, the LA85 strain involved in the present invention is named as *Lactobacillus acidophilus,* which was collected by the China General Microbiological Culture Collection Center on July 20, 2020, with a collection No. of CGMCC No.1.12735, in the following address: No. 3, Yard 1, Beichen West Road, Chaoyang District, Beijing.

According to the Classified Nomenclature, the Lp05 strain involved in the present invention is named as *Lactobacillus plantarum,* which was collected by the China General Microbiological Culture Collection Center on October 9, 2021, with a collection No. of CGMCC No.23547, in the following address: No. 3, Yard 1, Beichen West Road, Chaoyang District, Beijing.

### Description of Drawings

Fig. 1 shows the statistical results of white blood cell count in the bronchoalveolar lavage fluid of each group of mice 6 hours after infection with Pseudomonas aeruginosa.
Fig. 2 shows the statistical results of white blood cell count in the bronchoalveolar lavage fluid of each group of mice 24 hours after infection with Pseudomonas aeruginosa.
Fig. 3 shows the statistical results of Pseudomonas aeruginosa count in the lung tissue of each group of mice 24 hours after infection with Pseudomonas aeruginosa.

### Embodiments

The technical solution of the present invention will be further detailed below in embodiments. Those skilled in the art should appreciate that the examples described herein are only intended to facilitate understanding the present invention and should not be regarded as limitation on the present invention.

The formula of the medium involved in the following examples is as following:
MRS medium: peptone 10g/L, beef extract 10g/L, glucose 20g/L, sodium acetate 2g/L, yeast powder 5g/L, diammonium hydrogen citrate 2g/L, K₂PO₄·3H₂O 2.6g/L, MgSO₄·7H₂O 0.1g/L, MnSO₄ 0.05g/L, Tween 80 1mL/L, cysteine hydrochloride 0.5g/L.

According to the Classified Nomenclature, the LA85 strain involved in the following examples is named as *Lactobacillus acidophilus,* which was collected by the China General Microbiological Culture Collection Center on July 20, 2020, with a collection No. of CGMCC No.1.12735, in the following address: No. 3, Yard 1, Beichen West Road, Chaoyang District, Beijing.

According to the Classified Nomenclature, the Lp05 strain involved in the following examples is named as *Lactobacillus plantarum,* which was collected by the China General Microbiological Culture Collection Center on October 09, 2021, with a collection No. of CGMCC No.23547, in the following address: No. 3, Yard 1, Beichen West Road, Chaoyang District, Beijing.

Preparation method for the bacterial suspension mentioned below: inoculate the required strain in a liquid medium, and incubate at 37 °C for 24 hours for activation. An activate fluid is obtained after 2 times of continuous activation. Inoculate the activate fluid in the liquid medium at a dose of 5% (v/v), and incubate at 37 °C for 24 h to obtain a bacterial suspension. Centrifuge the bacterial suspension at 5,000rpm at 4 °C for 10min, filter to obtain the bacterial cells, and then resuspend with PBS solution, thus the bacterial suspension is obtained.

The test results data are statistically analyzed using ggplot2 in R language, **** represents p<0.0001, *** represents p<0.001, ** represents p<0.01, * represents p<0.05, and NS. represents no significant difference.

### Example 1

This example was to explore the ability of the compound probiotic to improve the symptoms of mouse model with infectious pneumonia:
(1) Test animals: healthy male ICR mice, age: 6 weeks (56 mice). These mice were raised in a controlled environment under a 12h light/dark cycle, room temperature: 21-23 °C, humidity: 40-60%. The animals could eat and drink freely.
(2) Animal grouping: after 1 week of adaptive feeding, the mice were randomly divided into 7 groups with 8 mice in each group: control group, model group, LA85 group (intervention with LA85 bacterial suspension), Lp05 group (intervention with Lp05 bacterial suspension), LA85+Lp05 group (combined intervention with LA85 bacterial suspension and Lp05 bacterial suspension), ATCC11975+Lp05 group (combined intervention with commercially available Lactobacillus acidophilus ATCC11975 bacterial suspension and Lp05 bacterial suspension), LA85+ ATCC8014 group (combined intervention with LA85 bacterial suspension and commercially available Lactobacillus plantarum ATCC8014 bacterial suspension).
(3) Animal modeling and intervention methods:
   For the control group and bacterial suspension intervention groups, 30 µL (15 µL per nostril) of 0.9% sterile normal saline (control group) or corresponding bacterial suspensions (viable count: 1×10⁹ CFU/animal) were injected in the nasal cavities of mice 18 hours before surgical anesthesia, and the model group was not treated. The mice in the bacterial suspension intervention groups and the model group received intraperitoneal injection of 7% chloral hydrate solution (10 mg/kg body weight) for anesthesia, and then were inoculated with 20 µL of Pseudomonas aeruginosa (ATCC9027) bacterial suspension (concentration: 1×10⁸ CFU/mL) in the nasal cavities (10 µL per nostril).
(4) Analysis of indicators:

### (4.1) Detection of white blood cells in bronchoalveolar lavage fluid:

In each group, 4 mice were anesthetized and sacrificed after 6 h and 24 h of infection respectively, and then in a sterile environment, bronchoalveolar lavage was performed through injection of 500 µL of 0.9% sterile normal saline via tracheal puncture, so as to collect the bronchial alveolar lavage liquid (BAL). The white blood cells in the bronchoalveolar lavage fluid were counted by a cell counter for each group.

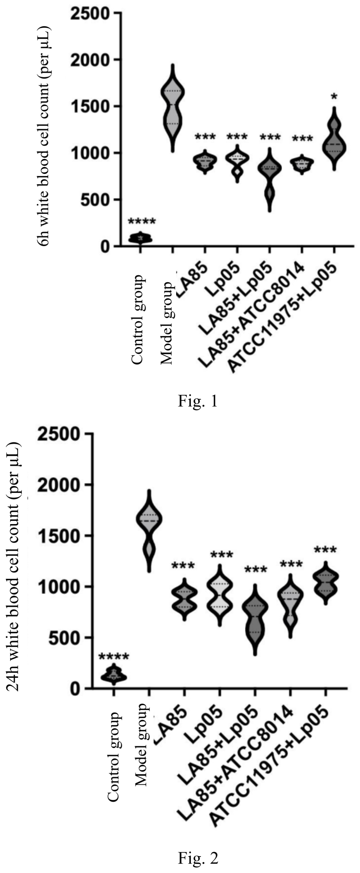

The results were shown in Figs. 1 and 2. As can be seen from the figures, the white blood cell count in the bronchoalveolar lavage fluid of the mice in the model group increased significantly as compared with the control group, indicating that the lungs of mice infected with Pseudomonas aeruginosa had significant inflammation, and thus, the model was successfully constructed. After the intervention of probiotics of each group, the white blood cell count in the bronchoalveolar lavage fluid of the mice decreased to varying degrees, in which the LA85+Lp05 group had the most significant decreasing trend and was superior to the Lp05 group and the LA85 group, indicating that the LA85 strain and Lp05 strain had a synergistic effect in alleviating infectious pneumonia.

### (4.2) Detection of pulmonary pathogenic bacteria:

The lungs of mice that had undergone 24h infection were excised, and homogenized with 0.9% sterile normal saline. The total lung homogenate on MRS agar plate was tested with the Organotial Mouse Lung Organoid Culture Medium Kit (Absin (Shanghai) Biotechnology Co., Ltd.), and the Pseudomonas aeruginosa and probiotics were counted by serial dilution. Incubate at 37 °C for 48 h under anaerobic conditions. The bacterial colony was determined using mass spectrometry, and the Pseudomonas aeruginosa in the total lung homogenate was counted. Take the logarithmic value.

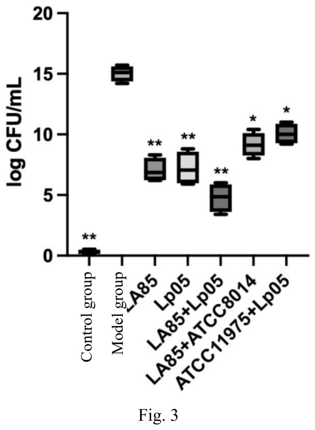

The results were shown in Fig. 3. As can be seen from the figure, the Pseudomonas aeruginosa count in the lung tissue of mice in the model group increased significantly as compared with the control group, indicating that serious Pseudomonas aeruginosa infection occurred in the lungs of mice in the model group. After the intervention of probiotics of each group, the Pseudomonas aeruginosa count in the lung tissue of the mice decreased to varying degrees, wherein the Pseudomonas aeruginosa count in the LA85+Lp05 group was significantly lower than that in the Lp05 group and the LA85 group, indicating that the LA85 strain and Lp05 strain had a synergistic effect in inhibiting the growth of Pseudomonas aeruginosa and alleviating infectious pneumonia.

### Example 2

This example was to explore the effect of the compound probiotic on the survival of mice with infectious pneumonia:
(1) Test animals: healthy male ICR mice, age: 6 weeks (35 mice). These mice were raised in a controlled environment under a 12h light/dark cycle, room temperature: 21-23 °C, humidity: 40-60%. The animals could eat and drink freely.
(2) Animal grouping: after 1 week of adaptive feeding, the mice were randomly divided into 7 groups with 5 mice in each group: control group, model group, LA85 group (intervention with LA85 bacterial suspension), Lp05 group (intervention with Lp05 bacterial suspension), LA85+Lp05 group (combined intervention with LA85 bacterial suspension and Lp05 bacterial suspension), ATCC11975+Lp05 group (combined intervention with commercially available Lactobacillus acidophilus ATCC11975 bacterial suspension and Lp05 bacterial suspension), LA85+ATCC8014 group (combined intervention with LA85 bacterial suspension and commercially available Lactobacillus plantarum ATCC8014 bacterial suspension).
(3) Animal modeling and intervention methods:
   For the control group and bacterial suspension intervention groups, 30 µL (15 µL per nostril) of 0.9% sterile normal saline (control group) or corresponding bacterial suspensions (viable count: 1×10⁹ CFU/animal) were injected in the nasal cavities of mice 18 hours before surgical anesthesia, and the model group was not treated. The mice in the bacterial suspension intervention groups and the model group received intraperitoneal injection of 7% chloral hydrate solution (10 mg/kg body weight) for anesthesia, and then were inoculated with 20 µL of Pseudomonas aeruginosa (ATCC9027) bacterial suspension (concentration: 1×10⁸ CFU/mL) in the nasal cavities (10 µL per nostril).
(4) Analysis of indicators:

### (4.1) Statistics on the survival of mice:

The mice were monitored within 7 days after infection with Pseudomonas aeruginosa, and the survival rate of mice in each group was calculated. The result was shown in the following Table 1:

**Table 1**

| | Survival rate | | | | | | |
|---|---|---|---|---|---|---|---|
| | Control group | Model group | LA85 group | Lp05 group | LA85+L p05 group | ATCC1197 5+Lp05 group | LA85+AT CC8014 group |
| 1 day | 100% | 100% | 100% | 100% | 100% | 100% | 100% |
| 2 days | 100% | 100% | 100% | 100% | 100% | 80% | 100% |
| 3 days | 100% | 80% | 100% | 100% | 100% | 80% | 100% |
| 4 days | 100% | 60% | 100% | 100% | 100% | 80% | 80% |
| 5 days | 100% | 60% | 100% | 100% | 100% | 60% | 80% |
| 6 days | 100% | 60% | 100% | 100% | 100% | 60% | 80% |
| 7 days | 100% | 40% | 100% | 80% | 100% | 60% | 80% |

As shown in Table 1, the compound probiotic involved in the present invention can significantly improve the survival of mice infected with Pseudomonas aeruginosa, and LA85+Lp05 group is superior to other groups intervened by bacterial suspensions, indicating that the LA85 strain and the Lp05 strain can cooperate with each other for mutual promotion, thus synergizing the effect of preventing and treating infectious pneumonia.

The applicant states: the technical solution of the present invention is illustrated through the above examples, but the present invention is not limited to the above examples, that is to say, the present invention can be implemented without relying on the above-mentioned examples. Those skilled in the art should appreciate that any improvement made to the present invention, equivalent replacement of raw materials of the products of the present invention and addition of auxiliary components, or selection of specific methods, etc. fall within the scope of protection and disclosure of the present invention.

The above provides a detailed description of the preferred embodiment of the present invention, however, the invention is not limited to the specific details mentioned in the above-mentioned embodiments. Those skilled in the art can make several simple modifications to the technical solution of the present invention, without departing from the spirit of the present invention. All those modifications shall be deemed as falling into the protection scope of the present invention.

In addition, it should be noted that the specific technical features described in above embodiments can be combined in any appropriate form, provided that there is no conflict. In order to avoid unnecessary repetition, the present invention will not elaborate on all possible combinations any further.

## Claims

1. A compound probiotic for prevention and treatment of infectious pneumonia, **characterized in that** the compound probiotic for prevention and treatment of infectious pneumonia consists of the *Lactobacillus acidophilus* LA85 strain with a collection No. of GMCC No.1.12735 and the *Lactobacillus plantarum* Lp05 strain with a collection No. of CGMCC No.23547.

2. The compound probiotic for prevention and treatment of infectious pneumonia according to claim 1, **characterized in that** the viable count ratio of the LA85 strain to the Lp05 strain is in a range of 1:10 to 10:1.

3. A probiotic preparation for prevention and treatment of infectious pneumonia, **characterized in that** the strains contained in the probiotic preparation include the compound probiotic according to claim 1 or 2.

4. The probiotic preparation according to claim 3, **characterized in that** the viable content of the LA85 strain and the Lp05 strain in the probiotic preparation is not less than 1×10⁹ CFU/g or 1×10⁹ CFU/mL respectively.

5. The probiotic preparation according to claim 3, **characterized in that** the dosage forms of the probiotic preparation include solution, freeze-dried powder, capsules, tablets or granules.

6. The probiotic preparation according to claim 5, **characterized in that** solution is adopted as the dosage form of the probiotic preparation, which is prepared by the following method:
inoculate the LA85 strain and Lp05 strain in the media respectively for activation and fermentation culture, so as to obtain the fermentation broths; centrifuge the fermentation broths respectively, and then resuspend with solvent to obtain LA85 bacterial suspension and Lp05 bacterial suspension; mix LA85 bacterial suspension and Lp05 bacterial suspension based on the viable count ratio to obtain the probiotic preparation.

7. The probiotic preparation according to claim 5, **characterized in that** freeze-dried powder is adopted as the dosage form of the probiotic preparation, which is prepared by the following method:
inoculate the LA85 strain and Lp05 strain in the media respectively for activation and fermentation culture, so as to obtain the fermentation broths; centrifuge the fermentation broths respectively, then mix with a protective agent and freeze-dry to obtain LA85 bacterial powder and Lp05 bacterial powder; mix LA85 bacterial powder and Lp05 bacterial powder based on the viable count ratio to obtain the probiotic preparation.

8. Use of compound probiotic according to claim 1 or 2 or probiotic preparation according to any one of claims 3 to 7 in preparation of drugs for prevention, alleviation or treatment of infectious pneumonia.

9. The use according to claim 8, **characterized in that**, the drug also contains excipients.

10. The use according to claim 9, **characterized in that**, said excipients include any one or at least two of fillers, binders, wetting agents, disintegrants, emulsifiers, co-solvents, solubilizers, osmotic pressure regulators, colorants, pH adjusters, antioxidants, bacteriostatic agents or buffers.
